# FASCICULE DE BREVET EUROPEEN

(11) **EP 2 923 196 B1**
(45) Date de publication et mention de la délivrance du brevet: **17.02.2021**
(21) Numéro de dépôt: 13803164.6
(22) Date de dépôt: 20.11.2013
(51) Int. Cl.: G01N 21/17, G01N 21/31, G01N 21/35, G01N 33/00, G01N 21/3504, G01J 3/42, G01N 21/47, G02B 26/08

(54) **DISPOSITIF DE DETECTION OPTIQUE DE GAZ A DISTANCE**
VORRICHTUNG ZUR OPTISCHEN FERNERKENNUNG VON GAS
DEVICE FOR THE REMOTE OPTICAL DETECTION OF GAS

(30) Priorité: 22.11.2012 FR 1261138
(43) Date de publication de la demande: 30.09.2015
(73) Titulaire: Bertin Technologies, 78180 Montigny-le-Bretonneux (FR)
(72) Inventeur: FERVEL, Franck, F-13710 Fuveau (FR); BERNASCOLLE, Philippe, F-83170 Tourves (FR)
(74) Mandataire: Decobert, Jean-Pascal
(86) Numéro de dépôt international: PCT/FR2013/052807
(87) Numéro de publication internationale: WO 2014/080127

(56) Documents cités:
- EP-A1- 0 544 962
- EP-A2- 0 287 929
- WO-A2-03/044499
- GB-A- 2 320 155
- ROBBERTO M ET AL: "Applications of DMDs for Astrophysical Research", OPTOMECHATRONIC MICRO/NANO DEVICES AND COMPONENTS III : 8 - 10 OCTOBER 2007, LAUSANNE, SWITZERLAND; [PROCEEDINGS OF SPIE , ISSN 0277-786X], SPIE, BELLINGHAM, WASH, vol. 7210, 1 January 2009 (2009-01-01), pages 72100A-1-72100A-9, XP007922732, DOI: 10.1117/12.809542 ISBN: 978-1-62841-730-2

## Description

L'invention concerne un dispositif de détection optique de gaz à distance, applicable notamment à la surveillance de sites industriels tels que des usines de produits chimiques, des raffineries, des installations de stockage de gaz, etc.

On connaît, par les documents EP-A-0544962 et WO 03/044499, un imageur infrarouge associé à des filtres optiques de mesure et de référence qui sont placés successivement sur l'axe optique de l'imageur et qui ont des bandes passantes contenant une raie d'absorption d'un gaz recherché (pour les filtres de mesure) ou qui sont complémentaires de cette raie d'absorption (pour les filtres de référence). On utilise le fond de la zone observée comme source infrarouge et on met en évidence la présence d'un gaz recherché par un traitement différentiel des images infrarouges prises à travers les filtres, le traitement permettant de calculer la concentration du gaz détecté.

De façon pratique, un ensemble de filtres de mesure et de référence est porté par un disque rotatif motorisé pour amener successivement les filtres sur l'axe optique de l'imageur. Les images de la zone observée dans les différentes bandes spectrales correspondant aux bandes passantes des filtres sont acquises séquentiellement.

Ce type de dispositif permet ainsi l'analyse et la recherche d'un gaz donné dans une zone d'espace en direction de laquelle est orienté l'imageur. Un tel dispositif nécessite une calibration préalable à l'aide d'un fond de scène émettant un flux radiatif standard sans gaz. Toutefois, ce type de calibration s'avère peu précis du fait de la difficulté à définir un fond standard, qui sera toujours différent du fond réel, ce qui limite fortement la précision des mesures de concentration en gaz. Ainsi, en pratique, ce type de dispositif sert essentiellement à identifier la présence d'un gaz donné mais ne permet pas d'indiquer avec précision sa concentration dans la zone d'espace observée. De plus, l'identification d'un gaz donné nécessite d'avoir un filtre de mesure avec une raie d'absorption correspondante, ce qui implique que l'analyse d'un mélange de gaz, comportant plusieurs raies d'absorptions différentes, est difficile à réaliser. Enfin, l'analyse des raies d'absorption des différents produits dans la zone observée est limitée par le nombre de filtres utilisés. Enfin, des produits différents peuvent présenter des raies d'absorption similaires.

La présente invention a notamment pour but d'éviter ces inconvénients d'une façon simple, efficace et économique.

A cet effet, elle propose un dispositif de détection optique d'un gaz, par exemple polluant, dans une zone d'espace observée, comprenant une caméra, un spectroscope infrarouge à transformée de Fourier et des moyens de détection en continu d'au moins un gaz dans tout ou partie de la zone observée à partir d'une analyse d'absorbance dans une pluralité de bandes spectrales différentes, caractérisé en ce qu'il comprend une matrice de micromiroirs individuellement orientables entre au moins deux positions dans une première desquelles ils renvoient le flux radiatif en provenance de la zone observée vers la caméra pour la détection de gaz dans lesdites bandes spectrales et une deuxième position dans laquelle ils renvoient le flux radiatif en provenance de la zone observée vers ledit spectroscope infrarouge à transformée de Fourier, et dans lequel la caméra comprend un système optique de prise d'images, des filtres et au moins un élément sensible sur lequel le système optique forme l'image renvoyée par la matrice de micromiroirs pour effectuer l'analyse d'absorbance, ladite caméra étant une caméra infrarouge ou un imageur infrarouge.

L'invention combine dans un seul et même dispositif des moyens de détection par analyse d'absorbance dans une pluralité de bandes spectrales et un spectroscope infrarouge à transformée de Fourier. Le couplage entre les sous ensembles du dispositif est réalisé au moyen de la matrice de micromiroirs permettant de renvoyer le flux radiatif de la zone observée vers la caméra ou vers le spectroscope à transformée de Fourier.

Un spectroscope comprenant une matrice de micromiroirs est utilisé par example dans l'observation de l'espace (Robberto, M. et al., "Applications of DMDs for Astrophysical Research", Proceedings of Spie/ IS&T, Vol. 7210).

L'utilisation d'une caméra à bandes spectrales permet une détection rapide en continu d'un ou de plusieurs gaz dans un large champ de vue et un spectroscope infrarouge à transformée de Fourier rapide permet une analyse spectrale précise du ou des gaz présents dans le flux radiatif d'une zone réduite de la scène observée, pour infirmer on confirmer la détection du gaz par la caméra.

Selon une autre caractéristique de l'invention, les moyens de détection de gaz comprennent au moins six bandes spectrales différentes pour la détection de gaz dans lesdites bandes spectrales.

La matrice de micromiroirs comprend un substrat sur lequel chaque micromiroir est articulé en rotation entre sa première et sa seconde position par l'intermédiaire de moyens d'application d'un champ électrostatique entre le substrat et le micromiroir.

Préférentiellement, les micromiroirs sont orientables chacun autour d'un pivot de manière à couvrir une étendue angulaire d'environ 24°.

L'invention concerne également un procédé de mise en œuvre du dispositif de détection décrit ci-dessus, caractérisé en ce qu'il consiste à :
a) commander l'orientation simultanée des micromiroirs de la matrice de manière à ce que tout le flux radiatif provenant de la zone d'espace observée soit orienté vers la caméra,
b) déduire la présence éventuelle d'un gaz dans tout ou partie de la zone d'espace observée à partir de l'analyse dans la pluralité de bandes spectrales,
c) en cas de détection positive d'un gaz dans une partie au moins de la zone observée, orienter au moins certains des micromiroirs correspondant à cette partie de la zone observée dans leur seconde position de manière à diriger une fraction du flux radiatif provenant de ladite partie vers le spectroscope infrarouge à transformée de Fourier,
d) confirmer ou infirmer la présence du gaz détecté à partir de l'analyse en transformée de Fourier.

La caméra collecte le flux radiatif en provenance de la zone observée et effectue une analyse par absorbance dans une pluralité de bandes spectrales dans les différentes directions comprises dans la scène observée.

En cas d'identification positive d'un gaz dans une partie de la scène observée, les miroirs de la matrice de micromiroirs correspondant à ladite partie d'espace sont orientés de manière à diriger le flux radiatif de cette partie d'espace vers le spectroscope infrarouge à transformée de Fourier rapide afin de réaliser une analyse spectrale précise du flux et infirmer ou confirmer la présence du gaz dans ladite partie d'espace.

D'autres avantages et caractéristiques de l'invention apparaîtront à la lecture de la description suivante faite à titre d'exemple non limitatif et en référence aux dessins annexés dans lesquels :
- la figure 1 est une représentation schématique du dispositif selon l'invention ;
- la figure 2 est une représentation schématique en perspective d'un micromiroir d'une matrice de micromiroirs utilisée dans l'invention ;
- la figure 3 représente très schématiquement l'allure d'une raie d'absorption d'un gaz ;
- les figures 4 et 5 représentent schématiquement, en fonction de la longueur d'onde, les bandes de transmission de deux filtres.

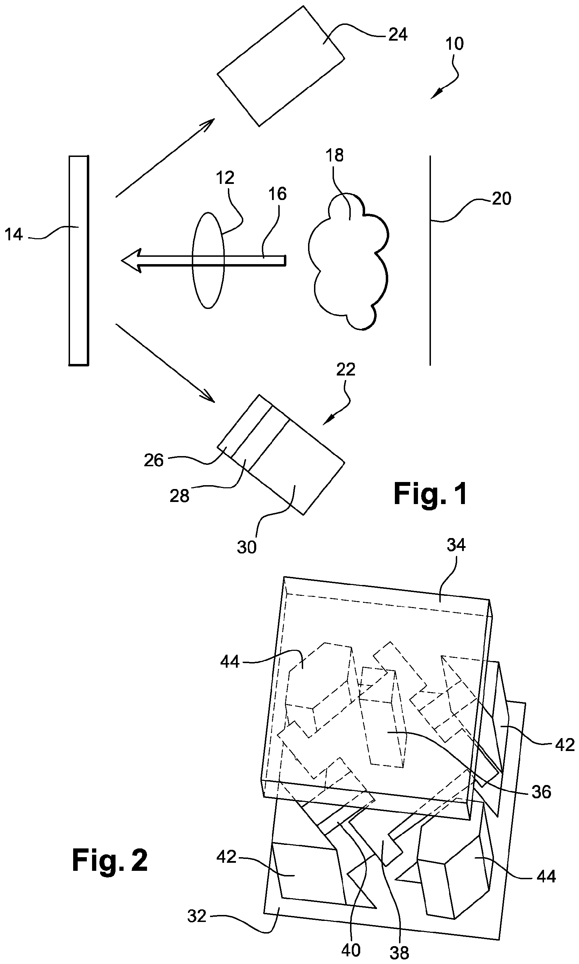

On se réfère tout d'abord à la figure 1 qui représente schématiquement un dispositif de détection 10 selon l'invention comprenant des moyens optiques 12 renvoyant sur une matrice de micromiroirs 14 le flux radiatif 16 d'une zone d'espace observée comportant par exemple un nuage d'un gaz 18 recherché et une scène de fond 20. Le dispositif comprend également une caméra ou imageur infrarouge 22 pour l'analyse d'absorbance dans une pluralité de bandes spectrales différentes du flux radiatif renvoyé par la matrice de micromiroirs 14 et un spectroscope infrarouge 24 à transformée de Fourier rapide.

La caméra comprend essentiellement un système optique 26 de prise d'images, des filtres 28 et au moins un élément sensible 30 sur lequel le système optique forme l'image renvoyée par la matrice de micromiroirs 14.

La matrice de micromiroirs 14 comprend une pluralité de micromiroirs portés par un même substrat 32 et individuellement orientables entre une première position dans laquelle ils renvoient le flux radiatif en provenance de la zone observée vers la caméra 22 et une deuxième position dans laquelle ils renvoient le flux radiatif vers le spectroscope infrarouge à transformée de Fourier 24 (figure 2).

La figure 2 représente la liaison d'un micromiroir 34 au substrat 32. Chaque micromiroir est relié par un poteau 36 à un plateau 38 monté sur un pivot 40 articulé en rotation à ses extrémités sur deux bras 42 du substrat 32. Le dispositif comprend des électrodes 44 d'application d'un champ électrostatique entre le substrat et le micromiroir de manière à faire pivoter le micromiroir 34 entre sa première et sa seconde position. Chaque pivot 40 peut comprendre un ressort de torsion (non représenté) monté autour du pivot 40 et solidaire à ses extrémités des bras 42 du substrat 32. Le ressort de torsion de chaque micromiroir est configuré de sorte que chaque micromiroir est maintenu dans sa première position par la force de torsion en l'absence d'application d'un champ électrostatique.

Ainsi, en l'absence d'application d'un champ électrostatique, les micromiroirs renvoi le flux radiatif vers la caméra 22. Lorsque l'on applique une force électrostatique à un micromiroir, celui-ci effectue une rotation de manière à basculer dans sa seconde position où il renvoie le flux radiatif vers le spectroscope à transformée de Fourier 24.

Les micromiroirs 34 sont ainsi individuellement orientables autour de leur pivot 40 de manière à couvrir par exemple une étendue angulaire d'environ -12° à +12° par rapport à un plan perpendiculaire au substrat 32.

Des matrices de micromiroirs de ce type sont commercialisées par de nombreuses entreprises et notamment par Texas Instruments.

Dans un mode de réalisation particulier, la caméra comprend au moins deux filtres 28 qui sont interposés séquentiellement et à tour de rôle ou en superposition sur l'axe optique de la caméra 22, au moyen d'un système motorisé.

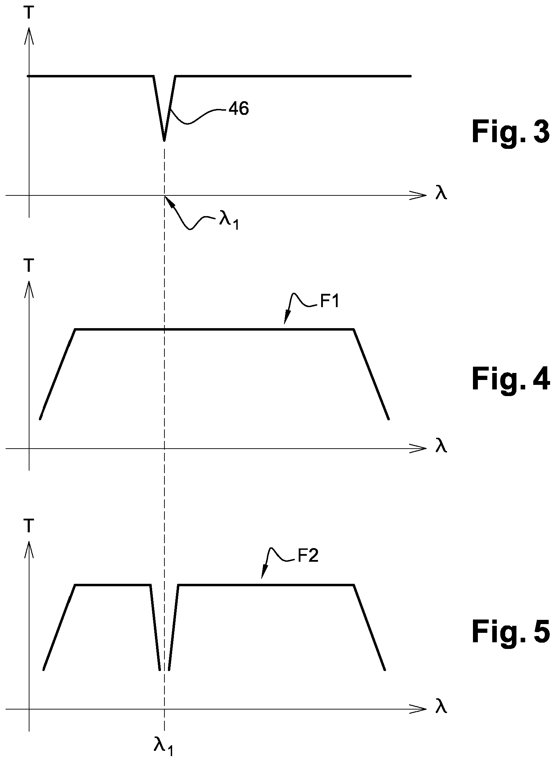

Ces deux filtres 28 ont des bandes de transmission en longueurs d'onde qui se recoupent en grande partie et qui sont de préférence globalement semblables, mais dont l'une inclut une raie d'absorption du gaz à détecter et dont l'autre est sensiblement complémentaire de cette raie d'absorption. Cette notion sera expliquée plus en détail en référence aux figures 3 à 5.

La figure 3 représente schématiquement la variation de la transmission T du gaz à détecter, dans une certaine bande de longueurs d'onde λ, la courbe de la transmission présentant une raie d'absorption 46 à une longueur d'onde λ₁, l'amplitude de cette raie d'absorption étant fonction de la concentration du gaz et sa largeur étant par exemple de l'ordre de quelques dizaines ou centaines de nanomètres.

La figure 4 représente schématiquement la courbe de transmission, en fonction de la longueur d'onde, d'un des deux filtres, par exemple un premier filtre que l'on désignera par la référence F1. Cette bande de transmission inclut la longueur d'onde λ₁ de la raie d'absorption du gaz à détecter, et s'étend sur une bande de longueurs d'onde qui est supérieure à la largeur de la raie d'absorption 46 du gaz à détecter.

L'autre filtre, que l'on désignera par la référence F2, a une bande de transmission dont l'allure est représentée en figure 5, qui ne comprend pas la longueur d'onde λ₁ de la raie d'absorption 46 du gaz à détecter et qui est en quelque sorte complémentaire de cette raie d'absorption par rapport à la bande de transmission du filtre F1 représentée en figure 4.

Ainsi, lorsque le filtre F1 est placé sur l'axe optique de la caméra ou de l'imageur 22, le flux radiatif reçu par l'élément sensible sera fonction de la présence ou de l'absence d'un nuage 18 du gaz à détecter dans la zone observée, et sera également fonction de la concentration de ce gaz.

Lorsque le filtre F2 est placé sur l'axe optique de la caméra ou de l'imageur thermique 22, le flux qu'il transmet à l'élément sensible est indépendant de la présence ou de l'absence d'un nuage 18 du gaz à détecter dans la zone observée.

Le rapport des flux fournis séquentiellement à l'élément sensible, à travers le filtre F1, puis à travers le filtre F2, fournit une grandeur qui est fonction de la concentration du gaz à détecter dans la zone observée, mais qui est indépendante de la température et de la chaine de transmission du flux radiatif à savoir, les moyens optiques 12, la matrice de micromiroirs 14 et les moyens optiques 26 de formation d'image de la caméra 22.

En variante, on peut placer sur l'axe optique le filtre F1 et faire les mesures, puis placer le filtre F2 sur l'axe optique en laissant en place le filtre F1, et refaire les mesures.

A l'aide de plusieurs ensembles de filtres ayant des raies d'absorption différentes comme décrit précédemment, il est possible de détecter en continu la présence de plusieurs gaz dans la zone d'espace observée.

Selon l'invention, la matrice de micromiroirs 14 est associée à un spectroscope infrarouge 24 permettant d'obtenir un interférogramme comprenant toutes les composantes fréquentielles d'une zone observée. Une transformée de Fourier rapide permet de visualiser des pics à différentes longueurs d'ondes correspondant à différents composés chimiques et d'en déduire précisément la présence de composés chimiques dans la zone observée avec une meilleure résolution spectrale que celle obtenue avec la caméra à bandes spectrales 22. La hauteur de chaque pic renseigne de manière précise sur la concentration du composé chimique relatif à ce pic.

Selon l'invention, le dispositif est mis en œuvre en commandant l'orientation simultanée des micromiroirs de la matrice 14 de manière à ce que tout le flux radiatif 16 provenant de la zone d'espace observée 20 soit dirigé vers la caméra. On effectue ensuite une analyse par absorbance dans une pluralité de bandes spectrales comme décrit précédemment pour déduire ou non la présence d'un composé gazeux prédéterminé auquel correspond une raie d'absorption. En cas de détection d'un gaz dans une partie de la zone observée, on sélectionne certains des micromiroirs de la matrice 14 correspondant à cette partie et on les oriente simultanément dans leur seconde position de manière à diriger au moins une fraction du flux radiatif provenant de cette partie vers le spectroscope infrarouge à transformée de Fourier pour confirmer ou infirmer la présence du gaz détecté à partir de l'analyse en transformée de Fourier. Lorsque certains micromiroirs sont positionnés dans leur seconde position, les autres micromiroirs continus à envoyer le flux radiatif vers la caméra 22, ce qui permet de faire un suivi en temps réel de la zone d'espace observée. Le dispositif selon l'invention peut être très compact et est réalisable sous forme d'un appareil mobile ou transportable, ce qui facilite les détections et les surveillances de sites.

Dans une réalisation pratique de l'invention, la caméra comprend entre six et neuf filtres et l'élément sensible 30 de la caméra est un capteur comprenant 640 par 480 pixels permettant de collecter en combinaison avec le système optique 26 un flux radiatif provenant d'une zone d'espace ouverte sur environ 30° dans la direction d'alignement des 640 pixels de la caméra et d'environ 24° dans la direction d'alignement des 480 pixels de la caméra.

Le spectroscope à transformée de Fourier est capable d'analyser une zone d'espace correspondant à un angle solide de 0,5° dans une direction par 0,5° dans une direction perpendiculaire. Ainsi, en pratique seuls quelques micromiroirs sont orientés de leur première position vers leur seconde position en cas de détection positive d'un gaz par la caméra 22. Dans une réalisation particulière de l'invention, la caméra comprend le même nombre de pixels que le nombre de micromiroirs.

Le dispositif selon l'invention combine une caméra à large ouverture angulaire de détection de gaz en temps réel mais qui ne permet pas de calculer précisément les concentrations en gaz détectés, avec un spectroscope à transformée de Fourier qui a une faible ouverture angulaire mais qui permet de détecter précisément les composés et leurs concentrations respectives dans une direction donnée de la zone d'espace analysée.

## Revendications

1. Dispositif de détection optique d'un gaz, par exemple polluant, dans une zone d'espace observée (20), comprenant une caméra (22), un spectroscope infrarouge à transformée de Fourier et des moyens de détection en continu d'au moins un gaz dans la zone observée à partir d'une analyse d'absorbance dans une pluralité de bandes spectrales différentes, **caractérisé en ce qu'**il comprend une matrice de micromiroirs (14) individuellement orientables entre au moins deux positions dans une première desquelles ils renvoient le flux radiatif (16) en provenance de la zone observée vers la caméra (22) pour la détection de gaz dans lesdites bandes spectrales et une deuxième position dans laquelle ils renvoient le flux radiatif (16) en provenance de la zone observée vers ledit spectroscope infrarouge à transformée de Fourier (24), et dans lequel la caméra comprend un système optique (26) de prise d'images, des filtres (28) et au moins un élément sensible (30) sur lequel le système optique (26) forme l'image renvoyée par la matrice de micromiroirs (14) pour effectuer l'analyse d'absorbance, ladite caméra étant une caméra infrarouge ou un imageur infrarouge.

2. Dispositif de détection selon la revendication 1, **caractérisé en ce que** les moyens de détection de gaz comprennent au moins six bandes spectrales différentes pour la détection de gaz dans lesdites bandes spectrales.

3. Dispositif selon la revendication 1 ou 2, **caractérisé en ce que** la matrice de micromiroirs comprend unsubstratsur lequel chaque micromiroir est articulé en rotation entre sa première et sa seconde position par l'intermédiaire de moyens d'application d'un champ électrostatique entre le substrat et le micromiroir.

4. Dispositif selon l'une des revendications 1 à 3, **caractérisé en ce que** les micromiroirs sont orientables chacun autour d'un pivot de manière à couvrir une étendue angulaire d'environ 24°.

5. Procédé de mise en œuvre du dispositif de détection selon l'une des revendications précédentes, **caractérisé en ce qu'**il consiste à :
a) commander l'orientation simultanée des micromiroirs de la matrice (14) de manière à ce que tout le flux radiatif (16) provenant de la zone d'espace observée soit orienté vers la caméra (22),
b) déduire la présence éventuelle d'un gaz dans une partie de la zone d'espace observée à partir de l'analyse dans la pluralité de bandes spectrales,
c) en cas de détection positive de la présence d'un gaz dans une partie de la zone observée, orienter au moins certains des micromiroirs correspondant à cette partie de la zone observée dans leur seconde position de manière à diriger une fraction du flux radiatif (16) provenant de ladite partie vers le spectroscope infrarouge à transformée de Fourier (24),
d) confirmer ou infirmer la présence du gaz détecté à partir de l'analyse en transformée de Fourier.

## Patentansprüche

1. Vorrichtung zur optischen Detektion eines z. B. umweltschädlichen Gases in einem überwachten Raumbereich (20), die eine Kamera (22), ein Fourier-Transformations-Infrarotspektroskop und Mittel zur kontinuierlichen Detektion mindestens eines Gases im überwachten Bereich auf Grundlage einer Absorptionsanalyse in einer Vielzahl verschiedener Spektralbänder umfasst, **dadurch gekennzeichnet, dass** sie eine Anordnung von Mikrospiegeln (14) umfasst, die einzeln zwischen mindestens zwei Stellungen ausgerichtet werden können, einer ersten, in der sie den aus dem überwachten Bereich kommenden Strahlungsfluss (16) für die Detektion von Gas in den Spektralbändern zur Kamera (22) umlenken, und einer zweiten Stellung, in der sie den aus dem überwachten Bereich kommenden Strahlungsfluss (16) zum Fourier-Transformations-Infrarotspektroskop (24) umlenken, und wobei die Kamera ein optisches System (26) zum Aufnehmen von Bildern, Filter (28) und mindestens ein empfindliches Element (30) umfasst, auf dem das optische System (26) das von der Mikrospiegelanordnung (14) umgelenkte Bild bildet, um die Absorptionsanalyse vorzunehmen, wobei es sich bei der Kamera um eine Infrarotkamera oder einen Infrarotbildaufnehmer handelt.

2. Detektionsvorrichtung nach Anspruch 1, **dadurch gekennzeichnet, dass** die Mittel zur Detektion von Gas mindestens sechs verschiedene Spektralbänder für die Detektion von Gas in diesen Spektralbändern umfassen.

3. Vorrichtung nach Anspruch 1 oder 2, **dadurch gekennzeichnet, dass** die Mikrospiegelanordnung ein Substrat umfasst, auf dem jeder Mikrospiegel mittels Mitteln zum Anlegen eines elektrostatischen Feldes zwischen dem Substrat und dem Mikrospiegel zwischen seiner ersten und seiner zweiten Stellung drehbar angelenkt ist.

4. Vorrichtung nach einem der Ansprüche 1 bis 3, **dadurch gekennzeichnet, dass** die Mikrospiegel jeweils um einen Drehpunkt ausgerichtet werden können, sodass sie einen Winkelbereich von etwa 24° abdecken.

5. Verfahren zum Einsetzen der Detektionsvorrichtung nach einem der vorstehenden Ansprüche, **dadurch gekennzeichnet, dass** es darin besteht:
a) die gleichzeitige Ausrichtung der Mikrospiegel der Anordnung (14) so zu steuern, dass der gesamte aus dem beobachteten Raumbereich kommende Strahlungsfluss (16) zur Kamera (22) ausgerichtet wird,
b) auf Grundlage der Analyse in der Vielzahl von Spektralbändern das eventuelle Vorhandensein eines Gases in einem Teil des überwachten Raumbereichs abzuleiten,
c) im Falle einer positiven Detektion des Vorhandenseins eines Gases in einem Teil des überwachten Bereichs mindestens gewisse der Mikrospiegel, die diesem Teil des überwachten Bereichs entsprechen, in ihre zweite Stellung auszurichten, um einen Bruchteil des Strahlungsflusses (16), der aus diesem Teil kommt, zum Fourier-Transformations-Infrarotspektroskop (24) zu lenken,
d) das Vorhandensein des detektierten Gases auf Grundlage der Fourier-Transformations-Analyse zu bestätigen oder zu widerlegen.

## Claims

1. A device for the optical detection of a gas, for example a pollutant, in an observed space area (20), comprising a camera (22), a Fourier transform infrared spectroscope and means for continuously detecting at least one gas in the observed area from an absorbance analysis in a plurality of different spectral bands, **characterised in that** it comprises a micromirror array (14) individually orientable between at least two positions in a first one of which they return the radiative flux (16) coming from the observed area towards the camera (22) for the detection of gas in said spectral bands and a second position wherein they return the radiative flux (16) coming from the observed area towards said Fourier transform infrared spectroscope (24), and wherein the camera comprises an imaging optical system (26), filters (28) and at least one sensitive element (30) on which the optical system (26) forms the image returned by the micromirror array (14) to perform the absorbance analysis, said camera being an infrared camera or an infrared imager.

2. The detection device according to claim 1, **characterised in that** the gas detection means comprise at least six different spectral bands for the detection of gas in said spectral bands.

3. The device according to claim 1 or 2, **characterised in that** the micromirror array comprises a substrate on which each micromirror is articulated in rotation between its first and its second position via means for applying an electrostatic field between the substrate and the micromirror.

4. The device according to one of claims 1 to 3, **characterised in that** the micromirrors are each orientable around a pivot so as to cover an angular extent of approximately 24°.

5. A method for implementing the detection device according to one of the preceding claims, **characterised in that** it consists in:
a) controlling the simultaneous orientation of the micromirrors of the array (14) so that all the radiative flux (16) coming from the observed space area is oriented towards the camera (22),
b) deducing the possible presence of a gas in a part of the observed space area from the analysis in the plurality of spectral bands,
c) in the event of positive detection of the presence of a gas in a part of the observed area, orienting at least some of the micromirrors corresponding to this part of the observed area in their second position so as to direct a fraction of the radiative flux (16) from said part to the Fourier transform infrared spectroscope (24),
d) confirming or denying the presence of the gas detected from the Fourier transform analysis.
